(19) Europäisches Patentamt
European Patent Office
Office européen des brevets

(11) Publication number: **0 003 892 A1**

# (12) EUROPEAN PATENT APPLICATION

(21) Application number: 79300237.9

(22) Date of filing: 15.02.79

(51) Int. Cl.²: C 07 D 499/00
A 61 K 31/43
//(C07D499/00, 277/00, 205/00)

(30) Priority: 16.02.78 GB 624178
16.02.78 GB 624278
08.03.78 GB 924878

(43) Date of publication of application:
05.09.79 Bulletin 79/18

(84) Designated contracting states:
BE CH DE FR GB NL SE

(71) Applicant: GLAXO GROUP LIMITED
Clarges House 6-12 Clarges Street
London W1Y 8DH(GB)

(72) Inventor: Cherry, Peter Clive
12 Thistledene Avenue
South Harrow Middlesex(GB)

(72) Inventor: Watson, Nigel Stephen
5 Ruislip Close
Greenford Middlesex(GB)

(72) Inventor: Evans, Derek Norman
17 Mount Park Road Ealing
London W5(GB)

(74) Representative: Holmes, Michael John et al,
Frank B. Dehn & Co. Imperial House 15-19 Kingsway
London WC2B 6UZ(GB)

(54) **Beta-lactam compounds, processes for their production, compositions containing them and intermediates of use in their preparation.**

(57) β-Lactam compounds of the formulae

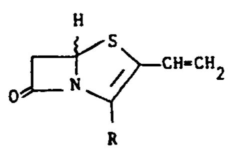

(IIIA)

and

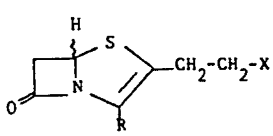

(IV)

are disclosed in which R represents a carboxyl or esterified carboxyl group and X represents a sulphonyloxy group. Processes for preparing the compounds and intermediates of use in their preparation are also disclosed.

The compounds are of value in the preparation of further β-lactam compounds, for example of formulae

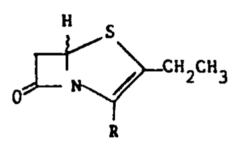

(XI) and

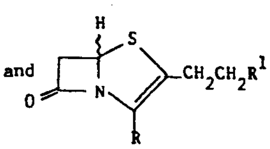

(I)

in which R is a carboxyl or esterified carboxyl group, and R' is the residue of a sulphur nucleophile.

Compounds of formula (I) are also disclosed, together with processes for their preparation, pharmaceutical compositions containing them and intermediates of use in their preparation. The compounds of formula (I) are of value as antibiotics or as β-lactamase inhibitors.

EP 0 003 892 A1

"β-Lactam compounds, processes for their production, compositions containing them and intermediates of use in their preparation"

This invention relates to novel penems having antibiotic activity, to processes for their production, to pharmaceutical compositions containing them and to intermediates of use in their preparation.

We have found that a new class of 6-unsubstituted penems, more particularly described below, possess useful antibiotic activity, and according to one aspect of the invention, we provide compounds of formula (I)

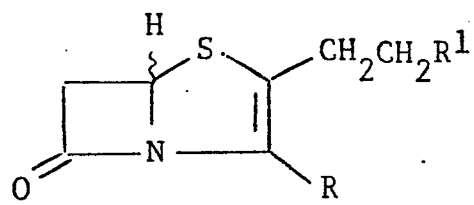

(I)

wherein R represents a carboxyl or esterified carboxyl group and $R^1$ represents the residue of a sulphur nucleophile, together with salts thereof.

The compounds of formula (I) may exist as individual 5-position isomers or as mixtures thereof and the invention extends to the isomers of the compounds individually or in admixture.

The carboxylic acid esters according to the invention may in general be represented as compounds of formula (I) in which R is a group $COOR^2$ where $R^2$ represents an organic group which is conveniently derived from an alcohol (aliphatic or araliphatic), a phenol, a

silanol or a stannanol. Such an alcohol, phenol, silanol or stannanol used to esterify the carboxyl group preferably contains not more than 24 carbon atoms.

Thus, the group $R^2$ may represent a straight or branched unsubstituted or substituted alkyl or alkenyl group preferably having from 1-8 carbon atoms, for example a methyl, ethyl, propyl, isopropyl, butyl, sec-butyl, tert-butyl or allyl group, optional substituents being for example, alkoxy e.g. methoxy; halogen i.e. fluorine, chlorine, bromine or iodine; cyano; acyloxy, e.g. alkanoyloxy such as acetoxy or pivaloyloxy or alkoxycarbonyloxy e.g. ethoxycarbonyloxy; acyl e.g. p-bromobenzoyl and alkoxycarbonyl e.g. ethoxycarbonyl;

an aralkyl group having up to 20 carbon atoms especially an arylmethyl group e.g. a benzyl or substituted benzyl group, suitable substituents being halo e.g. chloro; nitro e.g. o-nitro or p-nitro; cyano; alkoxy e.g. p-methoxy or alkyl e.g. p-methyl groups; a diphenylmethyl or triphenylmethyl group or a fur-2-ylmethyl, thien-2-ylmethyl or pyrid-4-ylmethyl group, the heterocyclic groups of which may also be substituted e.g. by a $C_{1-4}$ alkyl group, preferably methyl;

an aryl group having up to 12 carbon atoms, e.g. a phenyl or substituted phenyl group, suitable substituents being halo e.g. chloro; nitro e.g. o- or p-nitro; cyano; alkoxy e.g. p-methoxy or alkyl e.g. p-methyl groups;

a mono- or poly-cyclic cycloalkyl group containing not more than 12 carbon atoms, e.g. cyclohexyl, cyclopentyl or adamantyl;

a heterocyclic group containing not more than 12 carbon atoms, the heteroatom being, for example, oxygen

as in the tetrahydropyranyl or phthalidyl group;

a silyl group having up to 24 carbon atoms which may carry three groups which may be the same or different selected from alkyl, alkenyl, cycloalkyl, aralkyl and aryl groups. Such groups will preferably be $C_{1-4}$ alkyl e.g. methyl, ethyl, n-propyl, iso-propyl or t-butyl groups;

or a stannyl group having up to 24 carbon atoms for example a stannyl group carrying three substituents, which may be the same or different, selected from alkyl, alkenyl, aryl, aralkyl, cycloalkyl, alkoxy, aryloxy or aralkoxy groups. Such groups will include methyl, ethyl, propyl, n-butyl, phenyl and benzyl groups.

The group $R^1$ represents the residue of a sulphur nucleophile, for example an acylthio or thioacylthio group, a thioether group or a sulphone or sulphoxide derivative of said thioether group or a thiol group. In general these residues may be represented by the formulae $-SH$, $-SR^3$, $-SO.R^3$ or $-SO_2R^3$ (where $R^3$ is an aliphatic, araliphatic, aromatic or heterocyclic group) or by $-SC{=}Y.R^4$ (where Y is O or S and $R^4$ is a group as defined above for $R^3$ or a group $OR^3$ or $SR^3$, where $R^3$ is as defined above, or a group $NR^5R^6$, where $R^5$ and $R^6$, which may be the same or different, are hydrogen atoms or aliphatic, araliphatic, aromatic or carbon-attached heterocyclic groups or together with the nitrogen atom to which they are attached represent a heterocyclic ring).

Thus for example, $R^3$, $R^4$, $R^5$ and $R^6$ may be alkyl, alkenyl or alkynyl groups, which may contain 1-6 carbon atoms; aralkyl groups which may have 1-6 carbon atoms in the alkyl portion or aryl groups, such aryl and aralkyl

groups preferably being monocyclic; cycloalkyl groups, which may have 3-7, preferably 5 or 6 carbon atoms; or carbon-attached 5-7 membered heterocyclic rings containing one to four heteroatoms such as nitrogen, sulphur or oxygen, and may carry one or more alkyl groups which may have 1-6 carbon atoms. Such groups may themselves carry substituents such as hydroxyl or substituted hydroxyl, carboxyl or substituted carboxyl, amino or substituted amino, azido or cyano groups.

Representative groups $R^3$ include methyl, ethyl, propyl, butyl, allyl, propargyl, 2-aminoethyl, 2-azidoethyl, cyano-methyl, hydroxyethyl, ethoxyethyl, phenyl, benzyl, cyclohexyl, pyridyl, 1-methylpyridinium, 1-methyl-tetrazol-5-yl or 2-methyl-1,3,4-thiadiazol-5-yl.

Representative groups $R^4$.C=Y.- include ethoxythio-carbonyl, carbamoyl, thiocarbamoyl, dimethylthiocarbamoyl, thiobenzoyl, benzoyl, thioacetyl and acetyl. Where $NR^5R^6$ represents a heterocyclic ring, this may for example, contain 5-7 ring atoms, and may include one to three other heteroatoms e.g. nitrogen, oxygen or sulphur atoms, and may be, for example, a piperidino, piperazino, morpholino or thiamorpholino ring.

Substituted carboxyl groups may have the formula $COOR^7$, where $R^7$ is an aliphatic, araliphatic or aromatic group, while substituted amino groups may have the formula $NR^5R^6$ as defined above, one of $R^5$ and $R^6$ being other than hydrogen. Substituted hydroxy groups include acylated and etherified hydroxy groups $O.COR^7$ and $OR^7$ where $R^7$ is as defined above. The groups $R^5$, $R^6$ and $R^7$ may thu be $C_{1-4}$ alkyl groups e.g. methyl.

The salts according to the invention include carboxylate salts where R is carboxyl (including internal salts,e.g. zwitterions,when $R^1$ carries a basic group); also included are quaternary ammonium salts and acid addition salts of compounds in which $R^1$ carries a basic group $-NH_2$ or $-NR^5R^6$.

The carboxylate salts according to the invention include salts with inorganic bases, such as alkali metal salts e.g. sodium, potassium and lithium salts; alkaline earth metal salts e.g. calcium and magnesium salts; and ammonium salts, as well as salts with organic bases, for example substituted ammonium salts (e.g. salts with amines).

In general, the acids, salts and metabolically labile esters of the compounds of the invention are preferred forms for use in medicine. However, the other esters are also useful and, for example, where the ester grouping is readily cleaved e.g. by hydrolysis or hydrogenolysis, without significant degradation of the rest of the molecule, the esters are especially useful as carboxyl-protected derivatives of the parent compounds. Those esters which are readily cleaved and are primarily of use in this connection include arylmethyl esters, especially the benzyl, o- or p-nitrobenzyl, benzhydryl and trityl esters as well as the stannyl, e.g. tri-n-butyl-stannyl and silyl esters and 1-alkoxyalkyl e.g. methoxymethyl esters. As indicated above, the ester grouping may be metabolically labile, i.e. it may be converted into the carboxylic acid during general metabolic processes e.g. in the blood or liver. Metabolically labile esters include substituted alkyl esters carrying an oxygen substituent on the α-carbon atom for example α-acyloxyalkyl, e.g. acetoxymethyl and pivaloyloxymethyl, esters:

α-alkoxycarbonyloxyalkyl esters such as 1-ethoxycarbonyloxyethyl esters; and phthalidyl esters.

The acids, salts and metabolically labile esters of the compounds of formula (I) are generally active against a range of gram-positive and gram-negative microorganisms, for example against strains of Staphylococcus aureus, Micrococcus species, Escherichia coli, Salmonella typhimurium, Shigella sonnei, Enterobacter cloacae, Streptococcus faecalis, Klebsiella aerogenes, Proteus mirabilis, Serratia marcescens, Providence species and Haemophilus influenzae. The compounds have also been found stable to the action of β-lactamases produced by gram-positive organisms e.g. Staphylococcus aureus and gram-negative organisms e.g. Enterobacter cloacae, and further have the ability to inhibit β-lactamases produced by gram-negative organisms e.g. Enterobacter cloacae.

According to a further feature of the invention, we provide pharmaceutical compositions (including veterinary compositions) containing at least one of the acids, physiologically acceptable salts or metabolically labile esters of formula (I). The compositions may contain further β-lactam antibiotics. The compositions will normally also contain a pharmaceutical (including veterinary) carrier or excipient.

The compositions of the invention include those in a form adapted for oral or parenteral use.

The compositions may, for example, take the form of powders, tablets, capsules, lozenges, solutions and syrups suitable for oral administration, and may include, for example, starch, lactose, talc, magnesium stearate, gelatin, distilled water and suspending, dispersing, emulsifying, flavouring or colouring agents.

The compounds may further be formulated in rectal compositions such as suppositories or retention enemas.

The compounds of formula (I) may be formulated for parenteral administration e.g. for injection. The compounds may thus be formulated in ampoules for reconstitution before use.

The active compounds of the invention will generally be administered to humans at a total daily dosage level of 50 mg to 20 g, preferably from 100 mg to 10 g, advantageously 250 mg to 5 g, which may be in divided doses given 1-4 times per day. Dosage units will in general contain 12.5 mg to 5g, preferably 50 mg to 1 g of active compound according to the invention.

Active compounds of the invention may be of use in treating a variety of diseases in humans and animals, caused by pathogenic bacteria, such as respiratory tract or urinary tract infections.

The compounds of formula (I) may be prepared in a variety of ways. Thus, according to one feature of the invention, we provide a process for the preparation of a compound of formula (I) which comprises isomerising a compound of formula (II)

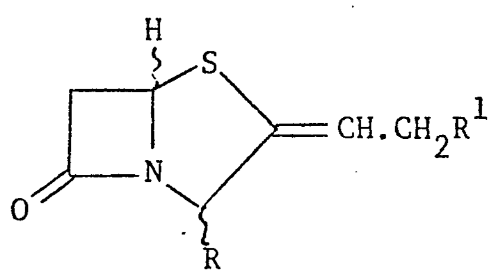

(II)

(wherein R and $R^1$ are as defined above) for example by treatment with a base whereby the compound of formula (I) is obtained, followed by deesterification and salt formation where required. In general, R is preferably an esterified carboxyl group.

The base used in the isomerisation will desirably have the formula $R^xR^yR^zN$ , where $R^x$, $R^y$ and $R^z$, which may be the same or different, may each represent an aliphatic, araliphatic or aromatic group e.g. alkyl groups having up to eight carbon atoms; aralkyl groups having up to six carbon atoms in the alkyl portion, or aryl groups, such aryl and aralkyl groups desirably being monocyclic, and including also cycloaliphatic e.g. $C_{3-7}$ cycloalkyl groups or compounds wherein two of $R^x$, $R^y$ and $R^z$ form, together with the nitrogen atom to which they are attached, a five-, six- or seven-membered heterocyclic ring optionally containing a further heteroatom e.g. an oxygen or sulphur atom as in N-alkyl piperidine or N-alkyl morpholine, or, wherein $R^x$, $R^y$ and $R^z$ form, together with the nitrogen atom to which they are attached, a monocyclic or polycyclic e.g. bicyclic heterocyclic ring system for example quinuclidine or 1,5-diazabicyclo[4.3.0]non-5-ene. Triethylamine or 1,5-diazabicyclo[4.3.0]non-5-ene are preferred bases. In general, the base used in the isomerisation should not result in any undesirable disruption of the bicyclic ring system of the starting material or product and it is preferred to use as weak a base as possible which is effective in the reaction.

This preference applies generally to those reactions described herein which involve use of a base.

Reaction will desirably be carried out in a suitable inert solvent. Such solvents will preferably have some degree of polarity and include esters e.g. ethyl acetate, ethers e.g. tetrahydrofuran, ketones e.g. acetone, amides e.g. dimethylformamide or halogenated hydrocarbons e.g. 1,2-dichloroethane or chloroform.

Reaction will desirably be effected at around ambient temperature, e.g. 0° to 30°C.

The compounds of formula (II), as defined above, together with salts thereof as defined above, are novel compounds and constitute another aspect of this invention.

The compounds of formula (II) may exist as individual 3-position and 5-position epimers as well as geometrical isomers with respect to the 2-position exocyclic double bond and the invention extends to all the isomers of the compounds individually or in admixture.

According to a further aspect of the invention, we provide a process for the preparation of compounds of formula (II) as defined above wherein a diene compound of formula (III)

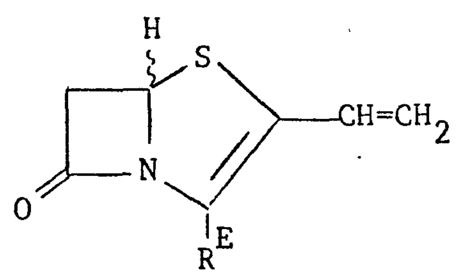

(III)

wherein $R^E$ is an esterified carboxyl group, is reacted with a sulphur nucleophile in the presence of a base or with a salt of the sulphur nucleophile and, where necessary a proton source, e.g. water, an alcohol or carboxylic acid e.g. acetic acid or ammonium chloride, whereby a compound of formula (II) is obtained, followed by deesterification and salt formation where required.

The sulphur nucleophile will in general be a compound of the formula $R^1.H$ where $R^1$ has the above meaning. Where a salt of the sulphur nucleophile is used, this may, for example, be an alkali metal (e.g. sodium, potassium or lithium), alkaline earth metal, ammonium or substituted ammonium salt.

Suitable bases for use in the reaction with the sulphur nucleophile include tertiary amines of the formula $NR^xR^yR^z$ as defined above.

Preferred organic bases include trialkylamines preferably having 1-6 carbon atoms in each alkyl group, especially methyl, ethyl, propyl or butyl groups, and triethylamine is convenient.

Other bases which may be used in the reaction with the sulphur nucleophile include alkali metal hydrides, alkoxides, dialkylamides, disilylamides, alkyls and aryls e.g. sodium, potassium or lithium hydrides or alkoxides or lithium dialkylamides, disilylamides, alkyls or aryls.

The above reaction will desirably be effected at or below ambient temperature, e.g. from -80°C to +40°C, e.g. from -20° to +20°C in solution in an inert solvent e.g. a halogenated hydrocarbon, such as dichloromethane; a substituted amide, such as dimethyl formamide; an ether ,

such as tetrahydrofuran; or an ester, such as ethyl acetate.

In a preferred embodiment of the reaction, however, the sulphur nucleophile in the presence of an inorganic base or a salt of the sulphur nucleophile may be reacted with the diene of formula (III) in a suitable aprotic liquid medium, e.g. a halogenated hydrocarbon solvent, containing a crown ether. A crown ether is a macrocyclic polyether, e.g. a number of ethyleneoxy units joined to form a ring, the internal diameter of the ring being approximately that of a particular metal ion. In general 6 ethyleneoxy units (18-crown-6) are suitable to entrain a potassium ion while 5 ethyleneoxy units (15-crown-5) are suitable for sodium. Some of the ethylene groups in such crown ethers may be replaced by other divalent units such as o-phenylene or 1,2-cyclohexylene units.

The inorganic base may, for example, be an alkali or alkaline earth metal hydroxide, carbonate or bicarbonate, e.g. potassium carbonate.

When one or more of the reactants, for example the base or the salt of the sulphur nucleophile, is not soluble in the solvent in which the reaction takes place, a phase transfer reagent, for example a crown ether, may also be used to effect transfer of the reactant or reactants from the solid phase to the liquid phase or between two liquid phases.

A preferred single-liquid-phase system is dichloromethane with potassium carbonate/18-crown-6. A preferred temperature range is 0° to 40°C.

Where $R^1$ represents an -SH group, the sulphur nucleophile may alternatively be a trithiocarbonate salt,

which generates trithiocarbonate ions as the sulphur nucleophile, followed by decomposition of the trithiocarbonate addition product, e.g. by using an acid such as hydrochloric acid, to yield the required -SH compound. The trithiocarbonate salt may, for example, be an alkali metal, alkaline earth metal or ammonium salt or a substituted ammonium salt.

Under certain conditions, particularly when using tertiary organic bases such as triethylamine or 1,5-diazabicyclo[4.3.0]non-5-ene, the compound of formula (II) initially produced may isomerise directly to a compound of formula (I). Where it is desired to isolate the compound of formula (II), it is preferred to use a crown ether with an inorganic base in the reaction with the sulphur nucleophile; however, where a tertiary organic base is used, it may be preferable to use a limited quantity of base, for example only a catalytic amount, and to isolate the compound of formula (II) as soon as possible after its formation.

Where direct conversion of a compound of formula (III) into a compound of formula (I) is desirable this may in general be achieved by using more vigorous reaction conditions than used for production of a compound of formula (II), e.g. a stronger base, a longer reaction time or a higher temperature or a combination of these. It will be appreciated that conversion of a compound of formula (III) into a compound of formula (II) involves the formation of a new chiral centre and the possibility of geometrical isomerism about the newly formed exocyclic double bond. Both epimers may be formed at the new chiral centre,

however those isomers having the hydrogen atoms at carbon atoms 3-and 5-in the penam ring system in the *cis*-configuration (i.e. the 3R, 5R and 3S, 5S isomers) tend to be unstable relative to the corresponding *trans*-isomers (i.e. the 3S, 5R and 3R, 5S isomers). Thus the 3,5 *trans*-isomers tend to be the major or sole products of the reaction. As the chirality of the carbon atom at the 3-position of the penam ring system is lost when the exocyclic double bond is moved into the ring to form the penem ring system (i.e. the conversion of (II) into (I)) the possibility of additional isomerism in compounds of formula (II) is of no consequence.

The initial product is generally an ester of formula (I) or formula (II) and where a different ester or a carboxylic acid product or salt thereof is required, the compound of formula (I) or formula (II) prepared may be deesterified and, if desired, reesterified by methods described below. Where an acid of formula (I) or formula (II) is to be prepared, it is preferred to employ a starting material, the ester group of which may subsequently readily be cleaved e.g. an arylmethyl ester, for example a benzyl, benzhydryl, trityl or *p*-nitrobenzyl ester.

Cleavage of such an arylmethyl ester, e.g. a *p*-nitrobenzyl ester, may be effected by hydrogenolysis for example using a metal catalyst, e.g. a noble metal such as platinum, palladium or rhodium. The catalyst may be

supported e.g. on charcoal or kieselguhr. A p-nitrobenzyl group may also be removed by reduction of the nitro group (e.g. using a dissolving metal reducing agent such as zinc in acetic acid, or zinc in aqueous tetrahydrofuran or acetone controlled, for example, in the pH range 3-6, preferably 4.0 - 5.5 by the addition of aqueous hydrochloric acid; aluminium amalgam in a moist ether, e.g. tetrahydrofuran; or iron and ammonium chloride in an aqueous ether e.g. tetrahydrofuran) followed by hydrolysis either under the reduction conditions or by subsequent treatment with acid. A p-nitrobenzyl ester can also be cleaved under alkaline conditions using, for example, sodium sulphide in a solvent such as aqueous tetrahydrofuran, dimethyl formamide or acetone. Alternatively, a stannyl or silyl ester can be cleaved by very mild solvolysis, e.g. by reaction with water, alcohols, phenols or carboxylic acids, e.g. acetic acid.

Where a salt of an acid of formula (I) or formula (II) is required, the acid may be reacted with a suitable base, solvents and reaction conditions preferably being chosen to favour precipitation of the desired salt. Thus, for example, in the formation of alkali metal salts, e.g. sodium or potassium salts, it is preferred to add to a solution of the acid in a solvent such as ethyl acetate, an alkali metal alkanoate, e.g. a 2-ethylhexanoate.

The resulting thio-compound may be isolated and purified by conventional techniques.

Where the initial product of formula (I) or (II) is a thiol ($R^1$=SH) the compounds in which $R^1$ is other than SH may be obtained therefrom by reaction with an alkylating or acylating agent.

The term "alkylating agent" is used herein also to include reagents serving to introduce substituted alkyl groups such as hydroxyalkyl, azidoalkyl, aminoalkyl, substituted aminoalkyl, cyanoalkyl, alkoxyalkyl and aralkyl groups as well as cycloalkyl groups or heterocyclic groups. Suitable alkylating agents include alkyl halides e.g. methyl or ethyl iodide, or benzyl bromide, or alkyl sulphates e.g. dimethyl sulphate; these reagents are reacted in the presence of a base. It is convenient to conduct the reaction using for example an inorganic base such as potassium carbonate in a halogenated hydrocarbon solvent e.g. dichloromethane, containing a crown ether. Also useful for introduction of substituted alkyl groups are alkyl or substituted alkyl vinyl ethers, e.g. ethyl vinyl ether, as well as cyclic vinyl ethers such as dihydropyran, or alkylenedialkyl ammonium salts such as methylenedimethylammonium iodide.

Suitable acylating agents include compounds of the formula $R^4$.C=Y.E where E is a readily eliminatable substituent e.g. halogen or acyloxy and $R^4$ and Y are as defined above. This reaction is desirably carried out in the presence of an acid binding agent e.g. pyridine.

The diene compounds of formula (III) and the parent acids having a carboxyl group in place of $R^E$ may be prepared by treatment of compounds of formula (IV)

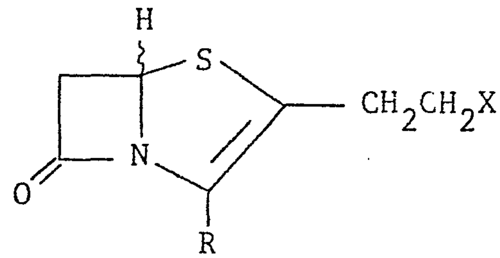

(IV)

wherein R is as defined above, preferably an esterified carboxyl group, and X is a readily eliminatable substituent, e.g. a sulphonyloxy group, with a base.

The base employed will preferably be a tertiary amine. Suitable amines will include compounds of the formula $NR^xR^yR^z$ as defined above.

Preferred bases include trialkylamines preferably having 1-6 carbon atoms in each alkyl group, especially methyl, ethyl, propyl or butyl groups, and triethylamine is particularly suitable. Pyridine may also be used.

Reaction will generally be effected in a suitable aprotic inert solvent. Such solvents will preferably have some degree of polarity and include esters e.g. ethyl acetate, ethers e.g. tetrahydrofuran or dioxan, ketones e.g. acetone, amides e.g. dimethylformamide, hydrocarbons e.g. benzene, halogenated hydrocarbons e.g. dichloromethane or 1,2-dichloroethane, sulphoxides e.g. dimethylsulphoxide or sulphones e.g. sulpholane.

Reaction may be effected at a low or moderate temperature e.g. a temperature in the range -30°C to +80°C. preferably from 0°C to 50°C.

As indicated above, the readily eliminatable substituent X may be a sulphonyloxy group, $-OSO_2R^A$, where the group $R^A$ represents an aliphatic, araliphatic or aromatic group. When $R^A$ is

an aliphatic group, it may represent a straight or branched unsubstituted or substituted alkyl or alkenyl group, preferably having from 1-8 carbon atoms, for example a methyl, ethyl, propyl or isopropyl, butyl, sec-butyl, tert-butyl or allyl group, optional substituents being for example, one or more alkoxy e.g. methoxy groups or halogen e.g. fluorine or chlorine atoms;

when $R^A$ represents an araliphatic group, it may represent an aralkyl group having up to 20 carbon atoms especially an arylmethyl group e.g. a benzyl or substituted benzyl group, suitable substituents being halo e.g. chloro; nitro e.g. o- or p-nitro; cyano; alkoxy e.g. p-methoxy or alkyl e.g. p-methyl groups;

when $R^A$ represents an aromatic group, it may represent an aryl group having up to 12 carbon atoms e.g. a phenyl or substituted phenyl group, suitable substituents being halo e.g. chloro; nitro e.g. o- or p-nitro; cyano; alkoxy e.g. p-methoxy or alkyl e.g. p-methyl groups.

Preferred groups $R^A$ include methyl, trifluoromethyl, phenyl or p-tolyl, the methyl and p-tolyl groups being especially preferred.

It may be convenient to convert the compound of formula (IV) into a compound of formula (I) or (II) without isolation of the diene of formula (III).

Introduction of a sulphonyloxy group as an eliminatable substituent X may readily be carried out by reaction of the compound of formula (V)

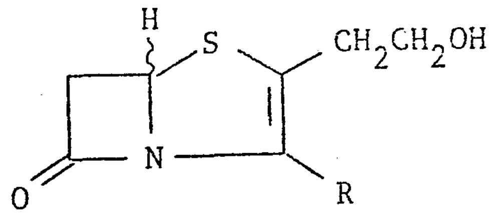

(V)

(wherein R is as defined above, preferably an esterified carboxyl group) with a hydrocarbon sulphonylating agent. The sulphonylating agent may, for example, be a sulphonyl halide or anhydrode $R^A.SO_2.Z$, wherein $R^A$ is as defined above and Z is a halogen, e.g. chlorine, bromine or iodine atom, or a sulphonyloxy group, preferably of the formula $-OSO_2R^A$.

Reaction will preferably be carried out in the presence of a hydrogen halide acceptor when a sulphonyl halide is employed as the sulphonylating agent. The hydrogen halide acceptor will preferably be a tertiary organic base, for example a compound of formula $NR^xR^yR^z$ as defined above, e.g. a tertiary amine such as a trialkylamine, especially triethylamine, or pyridine.

Where the sulphonyloxy derivative of formula (IV) is to be isolated a significant excess of the hydrogen halide acceptor should not be present, and the reaction will generally be effected at a temperature of from -80°C to +30°C, desirably -40° to 0°C.

Reaction will desirably be effected in an inert solvent, for example an ether e.g. tetrahydrofuran or dioxan, an ester e.g. ethyl acetate, a hydrocarbon e.g. benzene or a chlorinated hydrocarbon e.g. dichloromethane or 1,2-dichloroethane.

In view of the ease with which the sulphonyloxy compounds of formula (IV) may be converted into the diene, it may be convenient not to isolate the sulphonyloxy compound when it is formed but convert it into the corresponding diene compound of formula (III) in situ either by addition of further base, or by raising the temperature, or a combination of these methods. The base employed will preferably be a tertiary organic base e.g. a tertiary amine base $R^xR^yR^zN$ as defined above. Alternatively, the sulphonylation reaction can be carried out under conditions favouring elimination of the sulphonyloxy group initially introduced, for example at a temperature in the range 10° to 35°, and/or using an excess of base, for example a trialkylamine.

According to a further embodiment of the invention, the compounds of formula (I), other than those in which $R^1$ represents SH, may be prepared from compounds of formula (VI)

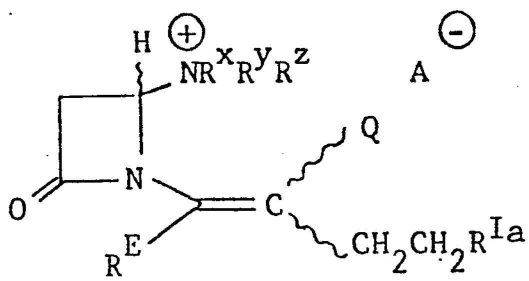

(VI)

(wherein $R^E$ represents an esterified carboxyl group, $R^{Ia}$ is the residue of a sulphur nucleophile other than SH, Q is a readily replaceable substituent, $R^x$, $R^y$ and $R^z$ are as defined above and $A^{\ominus}$ is an anion) by reaction thereof with hydrogen sulphide in the presence of a base or with a hydrosulphide or sulphide salt, followed, by heating to

effect cyclisation to the compound of formula (I) and, where a free acid or salt is desired, by deesterification and subsequent salt formation.

The base may be an inorganic or organic base. Preferred bases for use in the reaction are, for example, tertiary amine bases which may be of the formula $NR^xR^yR^z$ as defined above e.g. trialkylamines wherein the alkyl groups have e.g. 1-4 carbon atoms such as triethylamine or hindered bases e.g. 3-azabicylco[3.2.2]nonane. The sulphide or hydrosulphide salt may, for example, be an alkali metal or alkaline earth metal salt such as, e.g. a sodium, potassium or calcium salt or a quaternary ammonium salt.

The reaction with hydrogen sulphide or a salt thereof is preferably effected at a temperature in the range -30° to +30°. The cyclisation reaction is preferably effected at a temperature above 40°C for example in the range 50° to 100°C.

Reaction is preferably carried out in an inert liquid medium, which may be an inert solvent such as an ether, e.g. tetrahydrofuran or dioxan; an ester, e.g. ethylacetate or a chlorinated hydrocarbon, e.g. 1,2-dichloroethane.

The readily replaceable substituent Q in the compound of formula (VI) may for example, be a halogen (e.g. chlorine) atom or an acylated hydroxy group, such as an aliphatic, aromatic or araliphatic carbonyloxy, sulphonyloxy or phosphinyloxy group, containing for example 1-20 carbon atoms. The aliphatic or aromatic grouping of such a sulphonyloxy group may for example be an alkyl (e.g. $C_{1-8}$) group, which may be substituted by one or more halogen atoms e.g. fluorine or chlorine, or an aryl (e.g. $C_{6-15}$) group which may carry alkyl, e.g. methyl, alkoxy e.g. methoxy or halogen e.g. bromine substituents. The aliphatic or aromatic grouping of such a carbonyloxy group may be, for example, an alkyl group (e.g. $C_{1-8}$) optionally substituted by one or more halogen atoms, or an aryl (e.g. $C_{6-15}$) group, optionally substituted by for example, one or more halogen atoms or nitro groups.

Q is preferably the residue of an acid QH having a pKa in water at 25°C of 3.5 or less.

Preferred groups Q include mesyloxy, trifluoromethanesulphonyloxy, tosyloxy and phenylsulphonyloxy.

Q in the compounds of formula (VI) is conveniently mesyloxy.

$R^x$, $R^y$ and $R^z$ each preferably represent a $C_{1-6}$ alkyl group, especially a methyl, ethyl, propyl or butyl group; the triethylammonium group is particularly preferred.

Following the reaction with the hydrogen sulphide or sulphide salt it may be possible to isolate a thioenolate of the formula(VII)

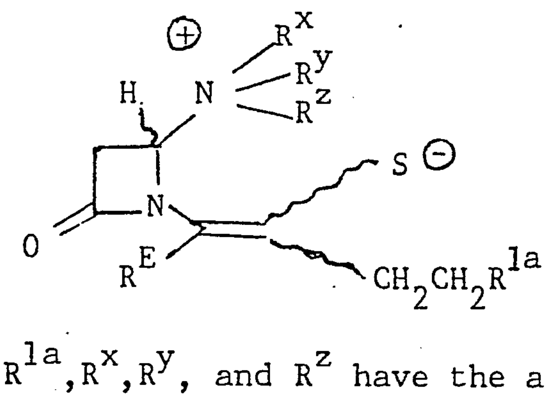

(VII)

(where $R^E$,$R^{1a}$,$R^x$,$R^y$, and $R^z$ have the above meanings). On heating,

e.g. in a solvent such as ethyl acetate or 1,2-dichloroethane, the compound of formula (VII) cyclises to the desired penem of formula (I).

The compounds of formulæ (VI) and (VII) as defined above are novel compounds and constitute a further feature of the invention.

The compounds of formula (VI) may be prepared from compounds of formula (VIII)

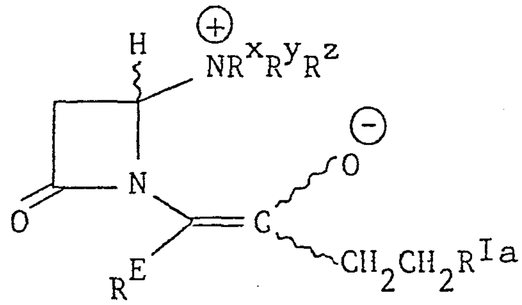

(VIII)

(wherein $R^{1a}$, $R^E$, $R^x$, $R^y$ and $R^z$ are as defined above by reaction with one or more reagents serving to introduce the readily replaceable substituent Q, for example by replacing the enolic hydroxyl group by a halogen atom or converting it into an acyloxy group.

The reagent serving to replace the hydroxyl group by a halogen atom may, for example, be a Vilsmeier reagent prepared from, for example, dimethylformamide and phosphorus trichloride.

Compounds of formula (VI) in which Q is an acyloxy group may be prepared by reacting a compound of formula (VIII) with an appropriate acylating agent, e.g. an acyl halide or anhydride. Such an acyl halide will desirably be a hydrocarbon sulphonyl halide wherein the hydrocarbon sulphonyl portion is as defined above. Thus the compound of formula (VIII) may be reacted with an alkane sulphonyl halide, for example methanesulphonyl halide or p-toluene

sulphonyl halide. Reaction is normally carried out in a solvent, e.g. an ether such as diethyl ether or tetrahydrofuran or a halogenated hydrocarbon such as methylene chloride or 1,2-dichloroethane.

The reaction will normally be effected in the presence of an acid binding agent, e.g. a tertiary amine which may have the formula $NR^xR^yR^z$ as defined above, such as triethylamine, pyridine or collidine, or an oxirane such as propylene oxide. If liquid, the acid binding agent may, if suitable, serve as a solvent. It is possible to convert a compound of formula (VIII) into the compound of formula (I) without isolation of any intermediate. Thus, for example, mesylation of a compound of formula (VIII) using mesyl chloride in pyridine may be followed by an _in situ_ cyclisation reaction by addition of an excess of triethylamine and passing in hydrogen sulphide; heating, e.g. to about 70°C, completes the reaction.

The initial product in the above reaction sequences is normally an ester of formula (I) and if an acid product or salt is required for further processing, the ester should subsequently be cleaved as described above.

Similarly, where an acid or salt of formula (II), (III) or (IV) is required and an ester is initially produced, such cleavage may be carried out, appropriate methods being selected to avoid unwanted reactions such as reduction of double bonds. If desired, such acids or salts may be re-esterified to introduce a different ester grouping.

Esters of the acids of formulae (I), (II), (III) or (IV) may be prepared by reaction of the acid with an alcohol, phenol, silanol or stannanol or a reactive derivative thereof

to form the desired ester. Reaction will desirably be effected under mild conditions in order to prevent rupture of the bicyclic nucleus. The use of neutral or mild acidic or basic conditions therefore, at temperatures between -70° and +35°C is preferred.

The alkyl, alkoxyalkyl and aralkyl esters may be prepared by reaction of the acid with the appropriate diazoalkane or diazoaralkane e.g. diazomethane or diphenyldiazomethane. The reaction will generally be effected in an ether, ester or a halohydrocarbon solvent, e.g. diethyl ether, ethyl acetate or dichloromethane. In general, reduced temperatures are preferred, for example, -15° to +15°C is preferred.

The esters derived from alcohols may also be produced by reaction of a reactive derivative of the alcohol, for example, a halide such as the chloride, bromide or iodide, or a hydrocarbonsulphonyl derivative such as a mesyl or tosyl ester, with a salt of the acid e.g. an alkali or alkaline earth metal salt such as a lithium, sodium, potassium, calcium or barium salt or an amine salt, such as triethylammonium salt. This reaction is preferably carried out in a substituted sulphoxide or amide solvent e.g. dimethyl sulphoxide, dimethylformamide or hexamethylphosphoramide. Alternatively, the esters may be prepared by reaction of the acid with the alcohol in the presence of a condensing agent such as dicyclohexylcarbodiimide.

Stannyl esters may conveniently be formed by reaction of the carboxylic acid or a salt thereof with reactive tetravalent tin moieties. Trialkyl tin oxides are preferred for the synthesis of tin compounds in view of their availability and low toxicity.

Silyl esters of the acid may be formed by reaction with a reactive tetravalent silicon moiety. Trialkylsilyl halides and mono- or bis-silylated acetamides are preferred as silylating agents. Proton acceptors e.g. weak bases such as pyridine may often be used with advantage when hydrogen halides are formed during such esterification.

The compounds of formula (V) whence compounds of the invention may be prepared, may be prepared by the methods described in our South African Patent No. 78/3986.

The compounds of formula (VIII) may be prepared by the methods described in our Belgian Patent No.858516.

According to a further feature of the invention, we provide vinyl compounds of formula (IIIA)

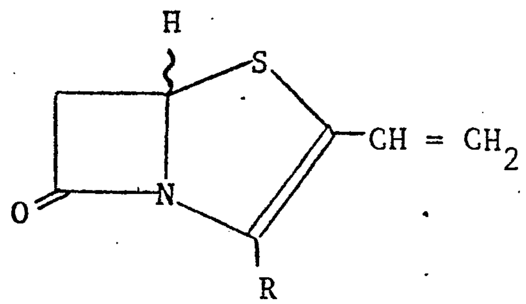

(IIIA)

in which R is a carboxyl or esterified carboxyl group, and salts thereof. These compounds are of use not only in the production of compounds of formula (I) as described above, but also in the production of 2-ethylpenems of the formula (IX)

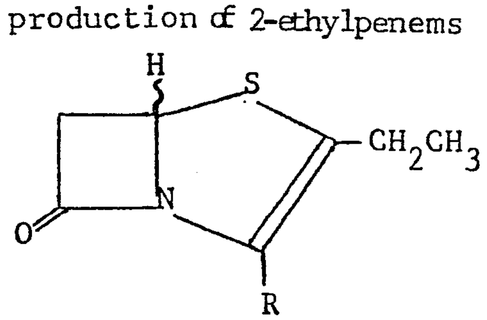

(IX)

described in our South African Patent No. 78/3986. Thus, the above 2-ethylpenems may be prepared from the compounds of formula (IIIA) by reduction of the vinyl group thereof to an ethyl group, e.g. by mild catalytic hydrogenation. Such

reduction constitutes a further feature of the invention.

The hydrogenation catalyst is normally a noble metal catalyst, e.g. palladium, platinum or rhodium. The catalyst may be supported e.g. on charcoal or kieselguhr; the metal catalyst is preferably palladium e.g. as 10% palladium on charcoal. Suitable solvents for the hydrogenation include ethers such as tetrahydrofuran; esters such as ethyl acetate; ketones such as acetone; amides such as dimethylformamide or halogenated hydrocarbons such as methylene chloride.

Where an ester of the diene is used as starting material and the ester grouping is susceptible to catalytic hydrogenolysis, the free acid of the 2-ethylpenem will be normally obtained. This may, if desired, be subjected to re-esterification or salt formation by the above methods.

The invention additionally provides compounds of formula (IV), where R and X have the above meanings, X being most preferably a group $O.SO_2R^A$ as defined above.

In the compounds of formula (IIIA) and (IV) the group R preferably represents an esterified carboxyl group $COOR^2$ in which $R_2$ is a readily cleavable group such as a benzyl, p-nitrobenzyl, o-nitrobenzyl or methoxymethyl group.

The invention also includes the sulphoxides of the compounds of formulae (I), (II), (IIIA) and (IV), wherein the ring sulphur atom is replaced by SO. These sulphoxides may be prepared from their corresponding sulphides by oxidation, for example using a peracid such as peracetic, monoperphthalic, m-chloroperbenzoic or metaperiodic acid preferably in the presence of an organic solvent such as acetic acid or dichloromethane. Preferred temperatures are not greater than 10°C e.g. 0°C. Under these conditions

a group $SR^3$ in compounds of formulae (I) and (II) will normally also be converted into a group $SOR^3$.

The invention will now be more particularly described by the following Examples and Preparations, which should not be taken as limiting the invention. Throughout the Examples and Preparations all temperatures are given in °C. The stereochemical designation (5RS) implies a racemic mixture of the (5R)- and (5S)- isomers. The designation (3SR,5RS,Z) implies a racemic mixture of the (3S,5R,Z)- and (3R,5S,Z)- isomers and the designation (3RS,5RS,Z) a racemic mixture of the (3R,5R,Z)- and (3S,5S,Z)- isomers.

i.e.

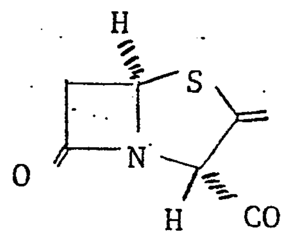
is 3S,5R

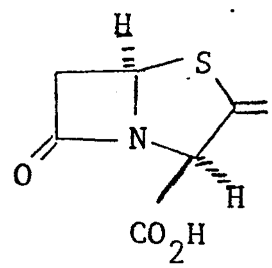
is 3R,5R

The bicyclic products in this specification are named with reference to "penam", the conventional name given to the parent heterocycle of formula (A)

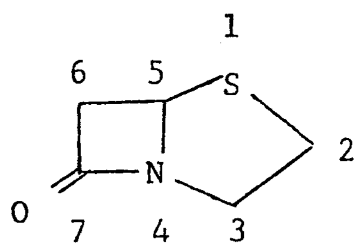
(A)

Example 1

4-Nitrobenzyl (5RS)-2-[2-(4-toluenesulphonyloxy)ethyl] pen-2-em-3-carboxylate

To a stirred solution/suspension of 4-nitrobenzyl (5RS)-2-(2-hydroxyethyl) pen-2-em-3-carboxylate (1.05 g; prepared as described in South African Patent No. 78/3986) in dry dichloromethane (40 ml) maintained at 0° and containing dry pyridine (0.260 g) was added 4-toluenesulphonic anhydride (1.077 g). After 2½ hours the reaction mixture was diluted with ethyl acetate (250 ml) and washed with water (2 x 100 ml). The organic phase was dried ($MgSO_4$), then evaporated to low volume, and silica gel was added. The slurry was evaporated to dryness and the impregnated silica gel was transferred to the top of a dry silica gel column and eluted with ethyl acetate - petroleum ether (40-60°) mixtures. Appropriate fractions were combined and evaporated to give an oil (0.935 g) which crystallised on trituration with ether to afford the title ester (0.725g), m.p. 98.0-101.0°, $\lambda_{max}$ ($CHCl_3$) 319 nm (ε 9180), $\nu_{max}$ ($CHBr_3$) 1786 (β-lactam), 1708 (ester), 1520 and 1346 $cm^{-1}$ ($NO_2$).

Example 2

4-Nitrobenzyl (5RS)-2-(2-trifluoromethanesulphonyloxyethyl) pen-2-em-3-carboxylate

To a stirred solution of 4-nitrobenzyl (5RS)-2-(2-hydroxyethyl)pen-2-em-3-carboxylate (0.105 g) in dry chloroform (4 ml) maintained at 0° and containing dry pyridine (0.06 ml) was added trifluoromethanesulphonic anhydride (0.06 ml). After 5 minutes the reaction

mixture was diluted with dry chloroform and washed with ice-cold water. The organic phase was dried ($MgSO_4$) and filtered to afford a chloroform solution of the <u>title ester</u> (0.085 g), $\nu_{max}$ ($CHCl_3$) 1800 (β-lactam), 1712 $cm^{-1}$ (ester), τ ($CDCl_3$) values include 4.32 (dd, J 4 and 2 Hz, C-5 H), 6.07 and 6.50 (dd, J 16 and 4 Hz; and dd, J 16 and 2 Hz, C-6 protons), 6.5 to 7.4 (m, $C\underline{H}_2CH_2O$).

<u>Example 3</u>

<u>4-Nitrobenzyl (5RS)-2-(2-methanesulphonyloxyethyl)pen-2-em-3-carboxylate</u>

A stirred suspension of 4-nitrobenzyl (5RS)-2-(2-hydroxyethyl)pen-2-em-3-carboxylate (1.0 g) in ethyl acetate (50 ml) at 0° was treated with methanesulphonyl chloride (0.36 ml) followed by triethylamine (0.63 ml). The suspension was stirred at 0° for 30 minutes and then partitioned between ethyl acetate and 0.5 N HCl. The organic phase was washed successively with brine, 0.5 N aqueous $NaHCO_3$ and brine. The resulting solution was dried over magnesium sulphate and evaporated to an oil which solidified on trituration with ether-petroleum spirit (1:1). The solid was collected, washed and dried <u>in vacuo</u> to give the <u>title ester</u> (1.176 g). A portion (180 mg) of this material was recrystallised from ethyl acetate-petroleum spirit to afford the <u>title ester</u> (123 mg), m.p. 103-104°, $\lambda_{max}$ ($CHCl_3$) 266 (ε 12,550) and 317.5 nm (ε 9,100).

Example 4

4-Nitrobenzyl (5RS)-2-(2-methanesulphonyloxyethyl)-pen-2-em-3-carboxylate 1-oxide

A stirred solution of the product of Example 3 (0.112 g) in dry dichloromethane (5 ml) was maintained at 5° and treated with m-chloroperbenzoic acid (0.053g). After 60 minutes the mixture was diluted with dichloromethane and washed with 0.5 N aqueous sodium hydrogen carbonate and brine. The dried organic solution was evaporated to afford the title ester, $\nu_{max}$ ($CHBr_3$) 1808 (β-lactam), τ($CDCl_3$) values include 5.03 (t, J 4 Hz, C-5H), 5.50 (t, J 7 Hz, $CH_2OSO_2$), 6.98 (s, $CH_3$).

Example 5

4-Nitrobenzyl (5RS)-2-vinylpen-2-em-3-carboxylate

A stirred suspension of 4-nitrobenzyl (5RS)-2-(2-hydroxyethyl)pen-2-em-3-carboxylate (0.15 g) in methylene chloride (8 ml) at 0° was treated with triethylamine (0.288 ml) followed by methanesulphonyl chloride (0.088 ml). The mixture was allowed to warm to ambient temperature and was stirred for 15 min. The resulting solution was absorbed on the top of a dry column of chromatographic silica gel which was then eluted with ether. Fractions were combined on the basis of t.l.c. examination and concentrated to ca. 3-4 ml whereupon crystallisation began. The crystalline solid was collected, washed with petroleum spirit and dried in vacuo to afford the title ester (0.079 g), m.p. 123-126° (Kofler), $\lambda_{max}$ (EtOH) 264 (ε 17,500) and 350.5 nm (ε 9,500).

Example 6

4-Nitrobenzyl (5RS)-2-vinylpen-2-em-3-carboylate

To a stirred solution/suspension of 4-nitrobenzyl (5RS)-2-(2-hydroxyethyl)pen-2-em-3-carboxylate (0.105 g) in dry dichloromethane (4 ml) maintained at 5° and containing dry pyridine (0.06 ml) was added trifluoromethanesulphonic anhydride (0.06 ml). After 5 minutes triethylamine (0.21 ml) was added and the reaction mixture stirred for a further 10 minutes, then diluted with ethyl acetate and washed successively with water, 0.5 N aqueous HCl, 0.5 N aqueous $NaHCO_3$, and saturated brine. The organic phase was dried ($MgSO_4$) and then evaporated to an oil which crystallised on trituration with ether to afford the title ester (0.02 g), whose spectroscopic properties resembled those as described in Example 5.

Example 7

4-Nitrobenzyl (5RS)-2-vinylpen-2-em-3-carboxylate

Triethylamine (0.101 g) was added to a stirred solution/suspension of the product of Example 1 (0.101 g) in ethyl acetate (5 ml). After 40 minutes the reaction mixture was diluted with ethyl acetate and washed successively with water, 0.5 N aqueous HCl, and saturated brine, and then dried ($MgSO_4$). Evaporation gave an oil which crystallised on trituration with ether toafford the title ester (0.05 g) having spectroscopic data similar to that of the product of Example 5.

Example 8

4-Nitrobenzyl (5RS)-2-vinylpen-2-em-3-carboxylate

A solution of the product of Example 3 (0.15 g) in ethyl acetate (7 ml) at 20° was treated with triethylamine (0.24 ml). After 30 minutes the mixture was diluted with ethyl acetate and washed with 0.5 N HCl followed by brine. The resulting solution was dried ($MgSO_4$) and evaporated to give an oil which solidified on trituration with ether-petroleum spirit (1:1). The solid was collected, washed and dried in vacuo to yield the title ester (0.076 g), whose properties resembled those described in Example 5.

Example 9

4-Nitrobenzyl (3SR,5RS,Z)-2-(2-acetylthioethylidene)-penam-3-carboxylate

A stirred solution of the product of Example 5 (0.74 g) in dichloromethane (30 ml) at ambient temperature was treated with 18-crown-6 (0.35 g), thioacetic acid (0.32 ml) and anhydrous $K_2CO_3$ (0.1 g). The mixture was stirred for 10 minutes, diluted with ether and washed successively with 0.5 N HCl, 0.5 N aqueous $NaHCO_3$ and brine. The dried organic solution was concentrated to give a slurry of crystals which were collected, washed with ether and dried in vacuo to afford the title ester (0.629 g) $\lambda_{max}$ (EtOH) 247 nm ($\varepsilon$ 16,300), $\nu_{max}$ ($CHBr_3$) 1780 ($\beta$-lactam), 1748 $cm^{-1}$ ($CO_2R$).

Example 10

Potassium (3SR,5RS,Z)-2-(2-acetylthioethylidene)penam-3-carboxylate

A solution of the product of Example 9 (0.568 g) in ethyl acetate (20 ml)-ethanol (20 ml) was hydrogenated at ambient temperature and atmospheric pressure over 10% palladium on carbon (2.25 g) for 30 minutes. The catalyst was removed by filtration through kieselguhr and the filtrate concentrated to ca. 10 ml. The resudue was stirred and treated with a solution of potassium 2-ethylhexanoate (0.19 g) in ethyl acetate (2 ml) followed by an excess of ether. The resulting precipitate was collected, washed with ether and dried *in vacuo* to give the title salt (0.258 g), $\lambda_{max}$ (pH 6 buffer) 235 nm (ε 10,300), $\nu_{max}$ (Nujol) 1727 (β-lactam) and 1634 $cm^{-1}$ ($CO_2^-$).

Example 11

4-Nitrobenzyl (3SR,5RS,Z)-2-(2-thiocarbamoylthioethylidene)penam-3-carboxylate

A stirred solution of the product of Example 5 (1.45 g) in dichloromethane (90 ml) at ambient temperature was treated with 18-crown-6 (0.75 g) and ammonium dithiocarbamate (1.5 g). After 2 hours the mixture was diluted with an equal volume of ethyl acetate and then concentrated to ca. half volume. The resulting solution was washed with water and brine, dried, and evaporated to dryness in the presence of chromatographic silica gel. The powder was added to the top of a dry column of silica gel and the column eluted with mixtures of ethyl acetate-petroleum spirit (1:3 to 1:1). Appropriate fractions

were combined and evaporated to a gum which solidified on trituration with petrol. The solid was collected, washed and dried to give the title ester (0.463 g), $\lambda_{max}$ ($CHCl_3$) 250 ($\varepsilon$ 19,900) and 275 nm ($\varepsilon$18,300), $\nu_{max}$ ($CHBr_3$) 3460 ($NH_2$), 1782 ($\beta$-lactam) and 1750 $cm^{-1}$ ($CO_2R$).

Example 12

Potassium (3SR,5RS,Z)-2-(2-thiocarbamoylthioethylidene)-penam-3-carboxylate

A suspension of the product of Example 11 (0.43 g) in ethyl acetate (30 ml)-tetrahydrofuran (20 ml) - ethanol (20 ml) was hydrogenated and worked up in the manner described in Example 10 to afford the title salt (0.064 g), $\lambda_{max}$ (pH 6 buffer) 275 nm ($\varepsilon$ 6,000), $\nu_{max}$ (Nujol) 1758 ($\beta$-lactam) and 1600 $cm^{-1}$ ($CO_2^-$).

Example 13

4-Nitrobenzyl (3SR,5RS,Z)-2-(2-methylthioethylidene)-penam-2-carboxylate

A stirred solution of the product of Example 5 (2.0 g) in dichloromethane (80 ml) at ambient temperature was treated with 18-crown-6 (1.0 g) and anhydrous $K_2CO_3$ (0.5 g). A stream of methane thiol was bubbled through the reaction mixture for 2 minutes. Further methane thiol was passed for ½ minute periods at intervals of 10 minutes up to 40 minutes. The reaction mixture was partitioned between ethyl acetate and water and the separated organic phase washed with water and brine. The dried solution was evaporated to an oil which was chromatographed on silica gel to yield the title ester (1.21 g), $\lambda_{max}$(EtOH) 261 nm ($\varepsilon$ 14,000), $\nu_{max}$ ($CHBr_3$) 1780 ($\beta$-lactam) and 1746 $cm^{-1}$ ($CO_2R$).

Example 14

Potassium (3SR,5RS,Z)-2-(2-methylthioethylidene)-penam-3-carboxylate

A solution of the product of Example 13 (0.72 g) in ethyl acetate (30 ml) - ethanol (30 ml) was hydrogenated and worked up in the manner described in Example 10 to afford the title salt (0.28 g), $\lambda_{max}$ (pH 6 buffer) 231 nm (ε 7,500), $\nu_{max}$ (Nujol) 1728 $cm^{-1}$ (β-lactam).

Example 15

4-Nitrobenzyl (3SR,5RS,Z)-2-(2-ethylthioethylidene)-penam-3-carboxylate

A stirred solution of the product of Example 5 (1.0 g) and 18-crown-6 (0.5 g) in dichloromethane (40 ml) at ambient temperature was treated with ethane thiol (0.75 ml) followed by anhydrous $K_2CO_3$ (0.25 g). After 10 minutes the mixture was partitioned between ethyl acetate and water. The organic phase was washed with water and brine and was then dried and evaporated to dryness in the presence of chromatographic silica gel. The resulting powder was added to the top of a dry column of silica gel and the column eluted with mixtures of ether and petroleum spirit. Appropriate fractions were combined and evaporated to give the title ester as an oil (0.996 g), $\lambda_{max}$ (EtOH) 261 nm (ε 12,900), $\nu_{max}$ ($CHBr_3$) 1780 (β-lactam) and 1745 $cm^{-1}$ ($CO_2R$).

Example 16

Potassium (3SR,5RS,Z)-2-(2-ethylthioethylidene)penam-3-carboxylate

A solution of the product of Example 15 (0.83 g) in ethyl acetate (30 ml) - ethanol (30 ml) was hydrogenated and worked up according to the method described in Example 10 to afford the title salt (0.253 g), $\lambda_{max}$ (pH 6 buffer) 231 nm ($\varepsilon$ 7,700), $\nu_{max}$ (Nujol) 1730 ($\beta$-lactam) and 1632 $cm^{-1}$ ($CO_2^-$).

Example 17

4-Nitrobenzyl (3SR,5RS,Z)-2-(2-phenylthioethylidene)-penam-3-carboxylate

A stirred solution of the product of Example 5 (1.0 g) in dichloromethane (40 ml) at ambient temperature was treated with 18-crown-6 (0.5 g), thiophenol (1.02 ml) and anhydrous $K_2CO_3$ (0.25 g). After 5 minutes the mixture was partitioned between ethyl acetate and water. The organic phase was washed with water and brine and then dried and evaporated to give an oil. Chromatography of the residue on silica gel with mixtures of ethyl acetate and petrol affored the title ester as an oil (0.875 g); $\lambda_{max}$ (EtOH) 253 nm ($\varepsilon$ 18,900), $\nu_{max}$ ($CHBr_3$) 1780 ($\beta$-lactam) and 1746 $cm^{-1}$ ($CO_2R$).

Example 18

Potassium (3SR,5RS,Z)-2-(2-phenylthioethylidene)-penam-3-carboxylate

A solution of the product of Example 17 (0.736 g) in ethyl acetate (30 ml) - ethanol (30 ml) was hydrogenated and worked up according to the method described in Example 10 to afford the title salt (0.275 g), $\lambda_{max}$ (pH 6 buffer) 237.5 nm ($\varepsilon$ 9,200), $\nu_{max}$ (Nujol) 1728 $cm^{-1}$ ($\beta$-lactam).

Example 19

4-Nitrobenzyl (3SR,5RS,Z)-2-(2-benzylthioethylidene)-penam-3-carboxylate

A stirred solution of the product of Example 5 (1.0 g) in dichloromethane (40 ml) at ambient temperature was treated with 18-crown-6 (0.5 g), benzyl thiol (0.35 ml) and anhydrous $K_2CO_3$ (0.25 g). After 10 minutes the mixture was partitioned between ethyl acetate and water. The organic solution was washed with water and brine and was then dried and evaporated to give an oil. Chromatography of the oil on silica gel with mixtures of ethyl acetate and petroleum spirit afforded the title ester (1.01 g), $\lambda_{max}$ (EtOH) 260.5 nm ($\varepsilon$ 13,600), $\nu_{max}$ ($CHBr_3$) 1780 ($\beta$-lactam) and 1742 $cm^{-1}$ ($CO_2R$).

Example 20

Potassium (3SR,5RS,Z)-2-(2-benzylthioethylidene)penam-3-carboxylate

A solution of the product of Example 19 (0.91 g) in ethyl acetate (30 ml) - ethanol (30 ml) was hydrogenated and worked up according to the method described in Example 10 to afford the title salt (0.27 g), $\nu_{max}$ (Nujol) 1730 ($\beta$-lactam) and 1636 $cm^{-1}$ ($CO_2^-$) $\tau(D_2O)$ values include 2.76 (s, Ph), 4.24 (t, J 8 Hz, =CH-), 4.86 (m, C-5 H)and 4.90 (s, C-3 H).

Example 21

4-Nitrobenzyl (3 SR,5RS,Z)-2-[2-(prop-1-en-3-yl)-thioethylidene]penam-3-carboxylate

A stirred solution of the product of Example 5 (1.0 g) in dichloromethane (40 ml) at ambient temperature was treated with 18-crown-6 (0.5 g), allyl thiol (0.24 ml)

and anhydrous $K_2CO_3$ (0.25 g). After 10 minutes the mixture was worked up in the manner described in Example 19 to afford the title ester (1.02 g), $\lambda_{max}$ (EtOH) 260 nm ($\varepsilon$ 12,700), $\nu_{max}$ ($CHBr_3$) 1780 ($\beta$-lactam) and 1744 $cm^{-1}$ ($CO_2R$).

Example 22

Potassium (3SR,5RS,Z)-2-[2-(prop-1-en-3-yl)thioethylidene]-penam-3-carboxylate

A solution of the product of Example 21 (0.88 g) in tetrahydrofuran (50 ml) and 0.5 N HCl (50 ml) was treated with zinc powder (1.15 g) and the mixture stirred for 45 minutes. The resulting mixture was partitioned between ethyl acetate and water. The organic solution was washed with water, dried and concentrated to ca. 10 ml. The residue was stirred and treated with a solution of potassium 2-ethylhexanoate (0.3 g) in ethyl acetate (10 ml) followed by ether. The precipitated solid was collected and partitioned between ethyl acetate and 0.5 N aqueous $NaHCO_3$. The aqueous phase was washed with ethyl acetate and then acidified with 2N HCl and extracted with ethyl acetate. The organic solution was dried and concentrated to ca. 10 ml. The residue was stirred and treated with potassium 2-ethylhexanoate (0.18 g) in ethyl acetate (10 ml). The precipitated solid was collected, washed and dried to yield the title salt (0.26 g), $\lambda_{max}$ (pH 6 buffer) 234 nm ($\varepsilon$ 8,000), $\nu_{max}$ (Nujol) 1730 ($\beta$-lactam) and 1638 $cm^{-1}$ ($CO_2^-$).

Example 23

4-Nitrobenzyl (3SR,5RS,Z)-2-(2-ethoxythiocarbonylthioethylidene)penam-3-carboxylate

A solution of the product of Example 5 (3.0 g) in dry dichloromethane (150 ml) was treated successively with 18-crown-6 (1.5 g), potassium ethylxanthate (3 g) and glacial acetic acid (0.56 ml). After 45 minutes, the solution was washed with water, 0.5 N HCl, 0.5 N aqueous $NaHCO_3$ solution and with brine. The dried organic solution was evaporated to afford a residue which was chromatographed on silica gel with ethyl acetate-petroleum spirit. Appropriate fractions were combined and evaporated to give a gum which was triturated with ether to afford the title ester as a solid (2.35 g), $\nu_{max}$ ($CHBr_3$) 1790 (β-lactam), 1758 ($CO_2R$), 1528 and 1351 $cm^{-1}$ ($NO_2$), τ($CDCl_3$) values include 4.60 (s, C-3 H), 4.70 (s, $C\underline{H}_2Ar$) 5.34 (q, J 7 Hz, $C\underline{H}_2CH_3$), 6.22 (d, J 7 Hz, $C\underline{H}_2SCS$), 8.58 (t, J 7 Hz, $CH_2C\underline{H}_3$).

Example 24

4-Nitrobenzyl (3SR,5RS,Z)-2-(2-N,N-dimethylthiocarbamoylthioethylidene)penam-3-carboxylate

A solution of the product of Example 5 (2.5 g) in dry dichloromethane (75 ml) was treated successively with sodium dimethyldithiocarbamate (2.0 g), 15-crown-5 (1 g) and acetic acid (0.44 ml). After 15 minutes the reaction mixture was washed with water, 0.5 N aqueous $NaHCO_3$ solution and brine. The dried organic solution was evaporated to afford the title ester as an oil (4.3 g), $\lambda_{max}$ ($CHCl_3$) 255 nm (ε 23,400), $\nu_{max}$ ($CHBr_3$) 1785 (β-lactam), 1750 ($CO_2R$) 1522 and 1346 $cm^{-1}$ ($NO_2$).

Example 25

4-Nitrobenzyl (3SR,5RS,Z) and (3RS,5RS,Z)-2-(2-benzoylthioethylidene)penam-3-carboxylate

A stirred solution of the product of Example 5 (0.38 g) in dry dichloromethane (20 ml) was treated successively with 18-crown-6 (0.2 g), a solution of thiobenzoic acid (0.5 g) in dichloromethane (10 ml), and $K_2CO_3$ (0.05 g). After 20 minutes the solution was washed with 0.5 N aqueous $NaHCO_3$ solution and water. The dried organic solution was evaporated to give an oil which was chromatographed on silica gel and eluted with mixtures of petroleum spirit and ethyl acetate to afford the (3SR,5RS,Z)-isomer (0.14 g), $\nu_{max}$ ($CHBr_3$) 1785 (β-lactam), 1750 ($CO_2R$), 1662 (SCOPh), 1524 and 1350 $cm^{-1}$ ($NO_2$), τ($CDCl_3$) values include 2.04 (m) and 2.4 - 2.7 (m)(Ph), 4.10 (t, J 8 Hz, =CH-) 4.60 (s, C-3H), 6.27 (d, J 8Hz, C<u>H</u>$_2$SCO).

Further elution afforded a mixture of isomers which was rechromatographed to give the (3RS,5RS,Z)-isomer (0.045 g), $\nu_{max}$ ($CHBr_3$) 1785 (β-lactam), 1750 ($CO_2R$), 1662 (SCOPh), 1522 and 1348 $cm^{-1}$ ($NO_2$), τ($CDCl_3$) values include 2.04 (m) and 2.3 - 2.6 (m) (Ph), 4.14 (t, J 8 Hz, =CH-), 5.40 (s, C-3H), 6.20 (d, J 8Hz, C<u>H</u>$_2$SCO).

Example 26

4-Nitrobenzyl (3SR,5RS,Z)-2-(2-acetylthioethylidene)-penam-3-carboxylate 1-oxide

A stirred solution of the product of Example 9 (1.44 g) in dry dichloromethane (100 ml) was maintained at 0° and treated with m-chloroperbenzoic acid (0.91 g) over 10 minutes. After a further 10 minutes the

reaction mixture was washed with 0.5 N aqueous $NaHCO_3$ solution and brine. The dried organic solution was evaporated to give the title ester (1.45 g), $\lambda_{max}$ ($CHCl_3$) 263 nm ($\varepsilon$ 11,000, $\nu_{max}$ ($CHBr_3$) 1790 (β-lactam) 1750 ($CO_2R$) and 1690 $cm^{-1}$ ($SCOCH_3$).

Example 27

Potassium (3SR,5RS,Z)-2-(2-acetylthioethylidene)penam-3-carboxylate 1-oxide

A solution of the product of Example 26 (1.84 g) in ethyl acetate (60 ml) - ethanol (60 ml) was hydrogenated at ambient temperature and atmospheric pressure for 105 minutes over 10% palladium on carbon (2.7 g). The catalyst was removed by filtration and the filtrate concentrated to ca. 15 ml. The residue was treated with a solution of potassium 2-ethylhexanoate in ethyl acetate (5 ml of 0.674 molar solution) followed by ether. The resulting precipitate was collected and partitioned between ethyl acetate and water. The separated aqueous phase was acidified with 2N hydrochloric acid and extracted with ethyl acetate. The dried ethyl acetate solution was concentrated to ca. 15 ml and treated with a solution of potassium 2-ethylhexanoate in ethyl acetate (4 ml of 0.674 molar solution). The resulting suspension was diluted with ether and the solid collected by filtration to give the title salt (0.57 g), $\nu_{max}$ (Nujol) 1775 (β-lactam), 1695 ($SCOCH_3$), 1625 ($CO_2^-$) and 1024 $cm^{-1}$ (S-O), $\tau$($D_2O$) values include 3.47 (m, =CH-), 4.8 - 5.1 (m, C-5 H and C-3 H), 7.62 (s, $SCOCH_3$).

Example 28

4-Nitrobenzyl (5RS)-2-(2-acetylthioethyl)pen-2-em-3-carboxylate

A solution of the product of Example 9 (0.7 g) in chloroform (25 ml) at ambient temperature was treated with 1,5-diazabicyclo[4.3.0]non-5-ene (DBN) (0.04 ml). After 45 minutes the mixture was diluted with ether and washed successively with water, 0.5 N HCl , water and brine. The dried organic solution was evaporated to an oil which crystallised on trituration with ether. The solid was collected, washed and dried to yield the title ester (0.518 g), $\lambda_{max}$ ($CHCl_3$) 265 (ε 13,100) and 320 nm (ε 8,100), $\nu_{max}$ ($CHBr_3$) 1790 (β-lactam), 1708 ($CO_2R$) and 1690$cm^{-1}$ ($SCOCH_3$).

Example 29

Potassium (5RS)-2-(2-acetylthioethyl)pen-2-em-3-carboxylate

A solution of the product of Example 28 (0.51 g) in ethyl acetate (30 ml) - ethanol (30 ml) was hydrogenated and worked up in the manner described in Example 10 to afford the title salt (0.132 g), $\lambda_{max}$ (pH 6 buffer) 302.5 nm (ε 5,100), $\nu_{max}$ (Nujol) 1755 (β-lactam), 1692 ($SCOCH_3$) and 1602 $cm^{-1}$ ($CO_2^-$).

Example 30

4-Nitrobenzyl (5RS)-2-(2-acetylthioethyl)pen-2-em-3-carboxylate

A stirred solution of the product of Example 5 (0.05 g) in dichloromethane (2 ml) at ambient temperature was treated with thioacetic acid (0.01 ml) followed by triethylamine (0.017 ml). After 30 minutes further portions of the thioacetic acid and triethylamine

were added and the mixture stirred for an additional 15 minutes. The solution was diluted with ether and washed successively with 0.5 N HCl, brine, 0.5 N aqueous $NaHCO_3$ and brine. The dried organic solution was evaporated to an oil which was redissolved in chloroform and evaporated to a gum which solidified on standing to give the title ester (0.065 g). The spectra of the product resembled those described in Example 28.

Example 31

4-Nitrobenzyl (5RS)-2-(2-methylthioethyl)pen-2-em-3-carboxylate

A solution of the product of Example 13 (0.43 g) in chloroform (20 ml) at ambient temperature was treated with DBN (0.05 ml) and stood for 6 hours. The solution was washed successively with 0.5 N HCl, 0.5 N aqueous $NaHCO_3$ and water. The dried organic solution was evaporated to an oil which was chromatographed on silica gel to give the title ester (0.24 g) $\lambda_{max}$ ($CHCl_3$) 265 (ε 12,900) and 315 nm (ε 7,000) $\nu_{max}$ ($CHBr_3$) 1785 (β-lactam) and 1708 $cm^{-1}$ ($CO_2R$).

Example 32

4-Nitrobenzyl (5RS)-2-(2-ethylthioethyl)pen-2-em-3-carboxylate

A solution of the product of Example 15 (0.04 g) in ethyl acetate (2 ml) at ambient temperature was treated with triethylamine (0.06 ml) and stood for 6 hours. The solution was diluted with chloroform and evaporated almost to dryness. The residue was redissolved in chloroform and evaporated to give the

title ester as an oil (0.039 g), $\nu_{max}$ ($CHBr_3$) 1790 (β-lactam) and 1710 $cm^{-1}$ ($CO_2R$), λ ($CDCl_3$) values include 4.35 (dd, J 2 and 4 Hz, C-5 H), 6.18 and 6.56 (dd, J 4 and 16 Hz, and dd, J 2 and 16 Hz, C-6 protons) and 8.74 (t, J 7 Hz, $CH_3$).

Example 33

4-Nitrobenzyl (5RS)-2-(2-phenylthioethyl)pen-2-em-3-carboxylate

A solution of the product of Example 17 (0.79 g) in chloroform (30 ml) at ambient temperature was treated with DBN (0.1 ml) and stood for 45 minutes. The resulting solution was washed successively with 0.5 N HCl, 0.5 N aqueous $NaHCO_3$ and water. The dried organic solution was evaporated to an oil which was chromatographed on silica gel with mixtures of ethyl acetate and petrol to give the title ester as an oil (0.37 g), $\lambda_{max}$ ($CHCl_3$) 258 (ε 18,300)and 320 nm (ε 7,400), $\nu_{max}$ ($CHBr_3$) 1778 (β-lactam) and 1708 $cm^{-1}$ ($CO_2R$).

Example 34

4-Nitrobenzyl (5RS)-2-(2-phenylthioethyl)pen-2-em-3-carboxylate

A solution of the product of Example 5 (2.35 g) in dichloromethane (150 ml) was treated with thiophenol (1.4 ml) and triethylamine (10 ml). After 1 hour at ambient temperature the solution was washed successively with 0.5 N HCl, 0.5 N aqueous $NaHCO_3$ and brine. The organic phase was dried ($MgSO_4$) and the solvent evaporated to afford an oil. The oil was chromatographed on silica

gel with mixtures of petroleum spirit (b.p. 40-60°) and ethyl acetate. Appropriate fractions were combined and evaporated to give the title ester as an oil (1.84 g). The spectroscopic properties of the product resembled those described in Example 33.

Example 35

4-Nitrobenzyl (5RS)-2-(2-ethoxythiocarbonylthioethyl)-pen-2-em-3-carboxylate

A solution of the product of Example 23 (2.28 g) and triethylamine (5 ml) in chloroform (150 ml) was stood at ambient temperature for 45 minutes. The reaction mixture was washed successively with 0.5 N HCl, 0.5 N aqueous $NaHCO_3$ and brine and the solution dried ($MgSO_4$). Evaporation of the solvent afforded the title ester as an oil (2.22 g), $\nu_{max}$ ($CHBr_3$) 1788 (β-lactam), 1708 ($CO_2R$), 1578 (C=C), 1520 and 1345 $cm^{-1}$ ($NO_2$), τ ($CDCl_3$) values include 4.36 (dd, J 4 and 2 Hz, C-5 H), 4.60 and 4.81 (ABq, J 15 Hz, $C\underline{H}_2Ar$), 5.39 (q, J 7 Hz, $C\underline{H}_2CH_3$), 8.61 (t, J 7 Hz, $CH_2C\underline{H}_3$).

Example 36

Potassium (5RS)-2-(2-ethoxythiocarbonylthioethyl)pen-2-em-3-carboxylate

A solution of the product of Example 35 (2.14 g) in ethyl acetate (100 ml) - ethanol (100 ml) was hydrogenated and worked up in a similar manner to that described in Example 27 to give the title salt (0.31 g), $\lambda_{max}$ (pH 6 buffer) 284 nm (ε 9,550), $\nu_{max}$ (Nujol) 1766 (β-lactam) and 1600 $cm^{-1}$ (carboxylate).

Example 37

4-Nitrobenzyl (5RS)-2-(2-N,N-dimethylthiocarbamoylthioethyl)pen-2-em-3-carboxylate

A solution of the product of Example 24 (4.1 g) and triethylamine (6 ml) in chloroform (50 ml) was stood at ambient temperature for 30 minutes and then washed successively with 0.5 N HCl, 0.5 N aqueous $NaHCO_3$ solution and brine. The dried organic solution was evaporated to give an oil which was chromatographed on silica gel and eluted with mixtures of petroleum spirit and ethyl acetate. Appropriate fractions were combined and evaporated to afford the title ester as a crystalline solid (1.65 g), $\lambda_{max}$ ($CHCl_3$) 255, 274 and 320 nm (ε19,600, 21,200 and 9,600) $\nu_{max}$ ($CHBr_3$) 1790 (β-lactam), 1708 ($CO_2R$), 1578 (C=C), 1520 and 1347 $cm^{-1}$ ($NO_2$).

Example 38

(5RS)-2-(2-N,N-Dimethylthiocarbamoylthioethyl)pen-2-em-3-carboxylic acid

A solution of 4-nitrobenzyl (5RS)-2-(2-N,N-dimethylthiocarbamoylthioethyl)pen-2-em-3-carboxylate (2.45 g) in ethyl acetate (150 ml) - ethanol (150 ml) was hydrogenated and worked up in a similar manner to that described in Example 27 to yield the title acid (0.48 g), $\lambda_{max}$ (pH 6 buffer) 256 nm (ε 14,100), $\nu_{max}$ (Nujol) 1790 (β-lactam), 1670 ($CO_2H$) and 1568 $cm^{-1}$ (C=C).

Example 39

4-Nitrobenzyl (5RS)-2-(2-benzoylthioethyl)pen-2-em-3-carboxylate

A solution of a mixture of the product of Example 25 (1.18 g) and triethylamine (1 ml) in chloroform (50 ml) was stood at ambient temperature for 90 minutes and then washed successively with 0.5 N HCl 0.5 N aqueous $NaHCO_3$ solution and brine. The dried organic solution was evaporated to give a gum which was triturated with ether and petroleum spirit to afford the title ester as a solid (0.92 g), $\nu_{max}$ ($CHBr_3$) 1794 (β-lactam), ($CO_2R$), 1666 (SCOPh), 1584 (C=C), 1524 and 1350 $cm^{-1}$ ($NO_2$), τ ($CDCl_3$) values include 2.06 (m) and 2.3 - 2.6 (m) (Ph), 4.31 (dd, J 4 and 2 Hz, C-5 H), 6.5 - 7.0 (m, $-C\underline{H}_2C\underline{H}_2-$).

Example 40

(5RS)-2-(2-Benzoylthioethyl)pen-2-em-3-carboxylic acid

A solution of the product of Example 39 (0.8 g) in ethyl acetate (80 ml) - ethanol (80 ml) was hydrogenated at ambient temperature and atmospheric pressure for 46 minutes over 10% palladium on carbon (4.0 g). The catalyst was removed by filtration through kieselguhr and the filtrate concentrated to ca. 10 ml). The residue was treated with a solution of potassium 2-ethylhexanoate in ethyl acetate (2.0 ml of 0.674 molar solution) and the precipitated material was collected to yield the potassium salt of the title acid. The product was partitioned between dilute hydrochloric acid and ethyl acetate. The dried organic phase was concentrated to ca.

1 ml and the separated solid was collected, washed and dried to afford the title acid (0.12 g) $\lambda_{max}$ (pH 6 buffer) 243 and 267 nm (ε 12,400 and 12,000), $\nu_{max}$ (Nujol) 1790 (β-lactam) and 1670 $cm^{-1}$ (SCOPh).

Example 41

4-Nitrobenzyl (5RS)-2-(2-benzoylthioethyl)pen-2-em-3-carboxylate

A solution of 4-nitrobenzyl (3SR,5RS,Z)-2-(2-benzoylthioethylidene)penam-3-carboxylate (0.935 g) and 1,5-diazabicyclo[4.3.0]non-5-ene (0.3 ml) was stood at ambient temperature for 30 minutes and then washed successively with 0.5 N HCl, 0.5 N aqueous $NaHCO_3$ solution and brine. The dried organic solution was evaporated to afford an oil which was chromatographed on silica gel and eluted with mixtures of petroleum spirit and ethyl acetate. Appropriate fractions were combined and evaporated to give the title ester as a solid (0.325 g). The spectroscopic properties of the product resembled those described in Example 39.

Example 42

(5RS)-2-Ethylpen-2-em-3-carboxylic acid

A solution of the product of Example 5 (0.1 g) in ethyl acetate (5 ml) was hydrogenated for 15 minutes at ambient temperature and atmospheric pressure over 10% palladium on carbon (0.8 g). The catalyst was removed by filtration through kiselguhr and the resulting solution evaporated to an oil which solidified on standing to afford the title acid (0.01 g, $\nu_{max}$, ($CHBr_3$) 1790 (β-lactam) and 1710 $cm^{-1}$ ($CO_2H$), τ ($CDCl_3$)

4.81 (dd, J 2 and 4 Hz, C-5 H), 6.11 and 6.52 (dd, J 4 and 17 Hz, and dd, J 2 and 17 Hz, C-6 protons), 6.8-7.2 (m, $\underline{CH_2}CH_3$) and 8.80 (t, J 7 Hz, $CH_2\underline{CH_3}$).

Example 43

Potassium 2-ethylpen-2-em-3-carboxylate

A solution of the product of Example 42 (0.6 g) in ethyl acetate (10 ml) - ethanol (100 ml) was hydrogenated for 30 minutes at ambient temperature and atmospheric pressure over 10% palladium on carbon (1.2 g). The catalyst was removed by filtration through kieselguhr and the resulting solution concentrated to *ca*. 10 ml. The residue was stirred and treated with a solution of potassium 2-ethylhexanoate (0.225 g) in ethyl acetate ( 5ml) followed by ether. The precipitated solid was collected, washed with ether and dried *in vacuo* to afford the *title salt* (0.05 g). The properties of the product resembled those described in Example 32 of South African Patent No. 78/3986.

The following Examples illustrate how compounds according to the invention maybe formulated into pharmaceutical compositions.

Example A

Formula per tablet

| | |
|---|---|
| Densified potassium (5RS)-2-(2-acetylthioethyl)pen-2-em-3-carboxylate containing 1% magnesium stearate | 302.0 mg |
| Empicol LZ | 4.5 mg |
| Explotab | 9.0 mg |
| Avicel PH 101 to tablet core weight of | 450.0 mg |

Method of Preparation

Blend the antibiotic with magnesium stearate and compress into slugs on a heavy duty tableting machine. Break down the slugs through a series of screens (10, 12, 16 and 20 mesh) on a rotary granulator to produce free-flowing granules. Blend the granules with the rest of the excipients and compress the blend on a tablet machine using normal or deep concave punches (9 - 12 mm diameter).

Film coat the tablet cores using hydroxypropylmethyl cellulose or similar film forming material using either an aqueous or non-aqueous solvent system. A plasticizer and suitable colour may be included in the film coating solution.

Example B

Dry Powder for Injection

Fill sterile potassium (5RS)-2-(2-acetylthioethyl)pen-2-em-3-carboxylate aseptically into glass vials under a blanket of sterile nitrogen such that each vial contains an amount equivalent to 500 mg of the antibiotic free acid. Close the vials using rubber discs or plugs held in position by aluminium sealing rings, thereby preventing gaseous exchange or ingress of microorganisms. Constitute the product by dissolving in Water for Injections shortly before administration.

Empicol LZ is sodium lauryl sulphate available from Marchon Ltd.; Explotab is sodium starch glycolate available from Greeff Fine Chemicals Ltd., Croydon, Surrey, England; Avicel PH 101 is microcrystalline cellulose available from FMC Corporation, U.S.A.

Results of Biological Tests

Biological testing of a number of compounds of the invention has provided the results shown below; the test compounds are identified by their Example Numbers:

1. Determination of Minimum Inhibitory Concentrations (MIC)

Serial two-fold dilutions of freshly prepared test solutions were made into Oxoid No. 1 Nutrient agar with or without added enrichment and poured into petri diches. Plates were inoculated with a multipoint inoculator with inoculum containing approximately $10^5$ colony forming units of the organisms shown in the following Tables, all of which organisms are clinical isolates. The MIC, quoted in the Tables as µg/ml, was read after 18 hours incubation at 37°C as the lowest concentration which inhibited growth.

| Organism \ Example No: | 29 | 36 | 38 |
|---|---|---|---|
| Staph. aureus 853E | <0.1 | <0.1 | <0.1 |
| Micrococcus Sp 1810E | <0.1 | <0.1 | <0.1 |
| E. coli 851E | 1 | 8 | 31 |
| E. cloacae 1051E | 16 | 62 | >250 |
| E. cloacae 1321E | 1 | 16 | 31 |
| K. aerogenes 1522E | 2 | 16 | 31 |
| Pr. mirabilis 431E | 1 | 8 | 16 |
| H. influenzae 1184E | 8 | 4 | 2 |

CLAIMS:-

1. Compounds of the formula (IIIA)

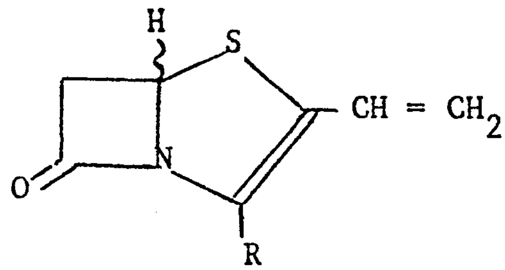

(IIIA)

where R is a carboxyl or esterified carboxyl group and salts thereof.

2. Compounds of the formula (IV)

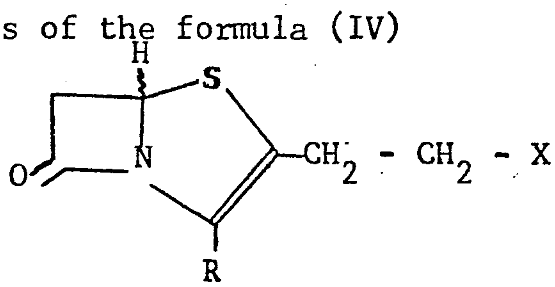

(IV)

(where R is a carboxyl or esterified carboxyl group and X is a sulphonyloxy group) and salts thereof.

3. Compounds as claimed in claim 2 in which X is a grouping of the formula $O.SO_2R^A$ , wherein $R^A$ is an aliphatic, araliphatic or aromatic group.

4. Compounds as claimed in any of claims 1-3 in which R is a group of the formula $COOR^2$ where $R^2$ is a straight or branched, substituted or unsubstituted alkyl or alkenyl group having from 1-8 carbon atoms; an aralkyl group having up to 20 carbon atoms; an aryl group having up to 12 carbon atoms, a cycloalkyl group having up to 12 carbon atoms, a heterocyclic group having up to 12 carbon atoms; or a silyl or stannyl group having up to 24 carbon atoms.

5. Compounds as claimed in any of claims 1-3 in which R is a benzyl or p-nitrobenzyl group.

6. A process for the preparation of a compound as claimed in claim 1 wherein a compound as claimed in claim 2 is reacted with a base.

7. A process as claimed in claim 6 in which the base is a tertiary amine.

8. A process for the production of a compound as claimed in claim 2 (wherein X is a group $O.SO_2R^{2A}$) in which a compound of formula (V)

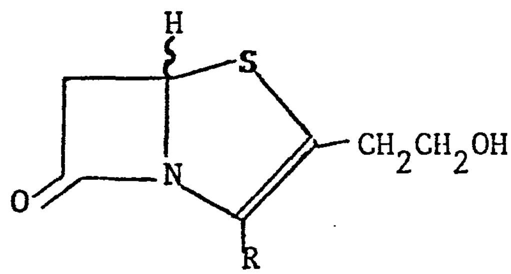

(V)

(where R is a carboxyl or esterified carboxyl group) or a salt thereof is reacted with a hydrocarbon sulphonylating agent serving to convert a hydroxyl group to a group $O.SO_2R^A$ (where $R^A$ is an aliphatic, araliphatic or aromatic group).

9. A process as claimed in claim 8 in which $R^A$ is a methyl, trifluoromethyl, phenyl or p-tolyl group.

10. A process as claimed in claim 8 or claim 9 in which the compound as claimed in claim 2 initially formed is permitted or caused to react *in situ* with a base whereby a compound as claimed in claim 1 is formed.

11. A process for the preparation of a 2-ethylpenem of the formula (XI)

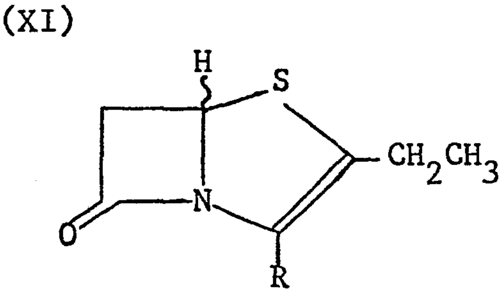

(XI)

(where R is a carboxyl or esterified carboxyl group) in which a compound as claimed in claim 1 is subjected to reduction whereby the vinyl group is reduced to an ethyl group.

12. A process for the production of a compound of the formula (I)

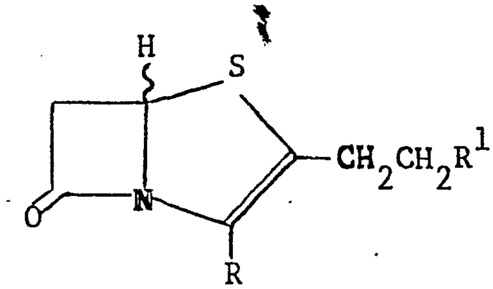

(I)

(where R is a carboxyl or esterified carboxyl group and $R^1$ is the residue of a sulphur nucleophile) or a salt thereof wherein a compound of the formula (II)

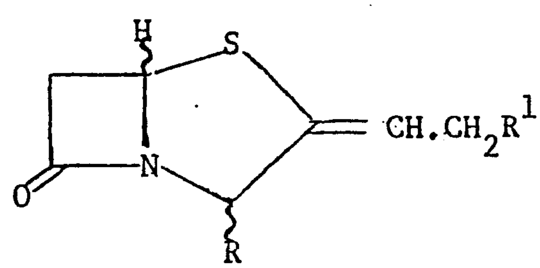

(II)

(where R and $R^1$ have the above meanings) or a salt thereof is subjected to isomerisation.

13. A process for the production of a compound of formula (II) as defined in claim 12 or a salt thereof wherein a compound of the formula (III)

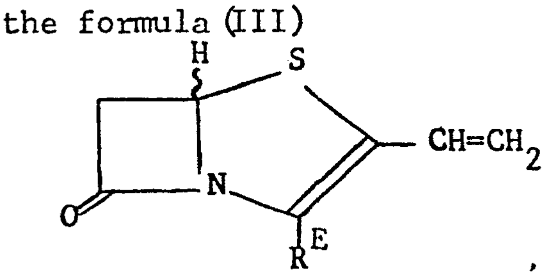

(III)

,

(where $R^E$ is an esterified carboxyl group) is reacted with a sulphur nucleophile in the presence of a base or with a salt of the sulphur nucleophile and, where necessary, a proton source, whereby a compound of formula (II) is formed, followed by deesterification and salt formation where required.

14. A process as claimed in claim 13 wherein the compound of formula (II) or salt thereof initially produced is isomerised in situ to a compound of formula (I) or a salt thereof.

15. Compounds of the formula (I) as defined in claim 12 and salts thereof.

16. Compounds as claimed in claim 15 in which $R^1$ is a group of the formula -SH, $-SOR^3$ or $-SO_2R^3$ (wherein $R^3$ is an aliphatic, aromatic, araliphatic or heterocyclic group) or $-SC{=}Y.R^4$ (where Y is O or S and $R^4$ is a group as defined above for $R^3$ or a group $OR^3$ or $SR^3$, where $R^3$ is as defined above, or a group $NR^5R^6$, where $R^5$ and $R^6$, which may be the same or different, are hydrogen atoms or aliphatic, araliphatic, aromatic or carbon-attached heterocyclic groups or together with the nitrogen atom to which they are attached represent a heterocyclic ring) or $-SR^{3A}$ (where $R^{3A}$ is a group as defined above for $R^3$ other than a lower alkyl group).

17. Compounds as claimed in claim 15 in which $R^1$ is a lower alkylthio group.

18. Compounds of the formula (II) as defined in claim 12.

19. A process for the preparation of compounds as claimed in claim 15 from compounds of formula VI)

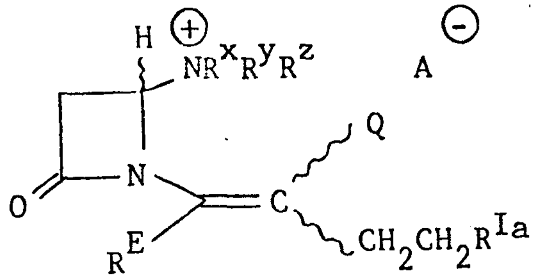

(VI)

(wherein $R^E$ represents an esterified carboxyl group, $R^I$ is the residue of a sulphur nucleophile other than SH, Q is a readily replaceable substituent, $R^x$, $R^y$ and $R^z$ are as defined above and $A^{\ominus}$ is an anion) by reaction thereof with hydrogen sulphide in the presence of a base or with a hydrosulphide or sulphide salt, followed, by heating to effect cyclisation to the compound of formula (I) and, where a free acid or salt is desired, by deesterification and subsequent salt formation.

20. Sulphoxides of the compounds of formulae (I), (II), (IIIA) and (IV) wherein the ring sulphur atom is replaced by SO.

21. Pharmaceutical compositions (including veterinary compositions) containing at least one of the acids, salts or metabolically labile esters as claimed in claim 15 together with a pharmaceutical (including veterinary) carrier or excipient and/or one or more further antibiotics.

0003892

European Patent Office

# EUROPEAN SEARCH REPORT

Application number
EP 79 30 0237

## DOCUMENTS CONSIDERED TO BE RELEVANT

| Category | Citation of document with indication, where appropriate, of relevant passages | Relevant to claim |
| --- | --- | --- |
| P | DE - A - 2 819 655 (CIBA-GEIGY)<br>* Claims 1-4,13,24-26 *<br>-- | 12,15-17,19-21 |
| P | EP - A - 0 000 636 (GLAXO)<br>* Claims 1-7 *<br>---- | 11,15-17,19-21 |

CLASSIFICATION OF THE APPLICATION (Int. Cl.³)

C 07 D 499/00
A 61 K 31/43//
(C 07 D 499/00
277/00
205/00)

TECHNICAL FIELDS SEARCHED (Int.Cl.³)

C 07 D 499/00
A 61 K 31/43

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
| --- | --- | --- |
| The Hague | 20-04-1979 | CHOULY |

EPO Form 1503.1 06.78